# EUROPEAN PATENT SPECIFICATION

(11) **EP 2 371 314 B1**
(45) Date of publication and mention of the grant of the patent: **08.05.2013**
(21) Application number: 11160184.5
(22) Date of filing: 29.03.2011
(51) Int. Cl.: A61B 18/12, A61B 18/14

(54) **Electrosurgical system with means for tracking the usage of a reposable instrument**
Elektrochirurgisches System mit Mitteln zur Verfolgung des Nutzens eines für eine begrenzte Anzahl von Nutzungen vorgesehenen Instruments
Système électrochirurgical avec des moyens pour suivre l'utilisation d'un instrument destiné à un nombre limité d'utilisations

(30) Priority: 29.03.2010 US 748526
(43) Date of publication of application: 05.10.2011
(73) Proprietor: Covidien LP, Mansfield, MA 02048 (US)
(72) Inventor: Gregg, William N., Superior, CO 80027 (US)
(74) Representative: HOFFMANN EITLE

(56) References cited:
- EP-A1- 1 535 583
- US-A1- 2007 083 111
- US-A1- 2007 167 720
- US-B1- 6 611 793

## Description

### BACKGROUND

### 1 Technical Field

The present disclosure relates generally to electrosurgical systems that utilize energy to perform electrosurgical procedures. More particularly, the present disclosure is directed to systems and apparatuses for tracking usage of reposable instrument devices.

### 2. Description of the Related Art

Energy-based tissue treatment is well known in the art. Various types of energy (e.g., electrical, ohmic, resistive, ultrasonic, microwave, cryogenic, laser) are applied to tissue to achieve a desired result. Electrosurgery involves application of high radio frequency electrical current to a surgical site to cut, ablate, coagulate or seal tissue. In monopolar electrosurgery, a source or active electrode delivers radio frequency energy from the electrosurgical generator to the tissue and a return electrode carries the current back to the generator. In bipolar electrosurgery, one of the electrodes of the hand-held instrument functions as the active electrode and the other as the return electrode. The return electrode is placed in close proximity to the active electrode such that an electrical circuit is formed between the two electrodes (e.g., electrosurgical forceps). In this manner, the applied electrical current is limited to the body tissue positioned between the electrodes.

Energy is supplied to the hand-held instrument or reposable instrument by an electrosurgical generator coupled thereto. A reposable instrument is a medical instrument limited to a specific number of uses or an instrument that is partly disposable and partly reusable. Because the reposable instrument has a limited window of use, it may be advantageous to know the usage and/or remaining life of the reposable instrument. Prior art reposable instruments were tracked by one or more systems or algorithms associated with the generator. However, such an arrangement required the reposable instrument to be paired with a specific generator. If the reposable instrument was used with multiple systems, tracking usage of the reposable instrument would be inaccurate.

Reference is made to US 6 611 793 B, which discloses a probe with a microprocessor adapted to determine whether the probe has been previously used and to disable a probe identification component based on the determination.

### SUMMARY

The invention is defined by the independent claim below dependent claims are directed to optional features and preferred embodiments.

An electrosurgical system is disclosed. The electrosurgical system includes a generator configured to output energy, an end effector operatively configured to output the energy to tissue, and a reposable instrument operatively coupled between the generator and the end effector. The reposable instrument includes a reposable instrument timing device configured to track usage of the reposable instrument, a controller configured to control operation of the electrosurgical system based on the usage of the reposable instrument, and a memory configured to store information pertaining to the usage of the reposable instrument.

The reposable instrument timing device provides reposable instrument usage information to the controller and the controller stores the reposable instrument usage information in the memory. The controller is configured to compare the reposable instrument usage information stored in the memory with a predetermined limit and if the reposable instrument usage information exceeds the predetermined limit, the controller disables the reposable instrument and may further provide a visual or audible indication to a user. The system may also include a transmission line configured to provide a timing signal from the generator to the reposable instrument and a transmission line configured to transfer data between the generator and the reposable instrument.

The electrosurgical system also includes an end effector timing device configured to track usage of the end effector in the reposable instrument. The end effector timing device provides end effector usage information to the controller and the controller stores the end effector usage information in the memory. The controller is configured to compare the end effector usage information stored in the memory with a predetermined limit and if the end effector usage information exceeds the predetermined limit, the controller disables the end effector and may further provide a visual or audible indication to a user.

A reposable instrument for use with an electrosurgical system is also disclosed. The reposable instrument may includes a reposable instrument timing device configured to track usage of the reposable instrument, a controller configured to control operation of the electrosurgical system based on the usage of the reposable instrument, and a memory configured to store information pertaining to the usage of the reposable instrument.

The reposable instrument timing device provides reposable instrument usage information to the controller and the controller stores the reposable instrument usage information in the memory. The controller is configured to compare the reposable instrument usage information stored in the memory with a predetermined limit and if the reposable instrument usage information exceeds the predetermined limit, the controller disables the reposable instrument and may further provide a visual or audible indication to a user.

The reposable instrument also includes an end effector timing device configured to track usage of the end effector. The end effector timing device provides end effector usage information to the controller and the controller stores the end effector usage information in the memory. The controller is configured to compare the end effector usage information stored in the memory with a predetermined limit and if the end effector usage information exceeds the predetermined limit, the controller disables the end effector or and may further provide a visual or audible indication to a user.

A portable electrosurgical device is also disclosed. The portable electrosurgical device includes an end effector operatively configured to output energy to tissue and a reposable instrument operatively coupled to the end effector. The end effector of the reposable instrument is adapted to connect to a generator The reposable instrument includes a reposable instrument timing device configured to track usage of the reposable instrument, a controller configured to control operation of the electrosurgical system based on the usage of the reposable instrument and a memory configured to store information pertaining to the usage of the reposable instrument.

### BRIEF DESCRIPTION OF THE DRAWING

The above and other aspects, features, and advantages of the present disclosure will become more apparent in light of the following detailed description when taken in conjunction with the accompanying drawings in which:

Fig. 1 is a schematic block diagram of an electrosurgical system for use with various instrument types;

Fig. 2 is a schematic block diagram of an electrosurgical system for use with various instrument types;

Fig. 3 is a schematic block diagram of an electrosurgical system according to an embodiment the present invention for use with various instrument types; and

Fig. 4 is a schematic block diagram of an electrosurgical system for use with various instrument types.

### DETAILED DESCRIPTION

Particular embodiments of the present disclosure are described hereinbelow with reference to the accompanying drawings; however, it is to be understood that the disclosed embodiments are merely exemplary of the disclosure that may be embodied in various forms. Well-known functions or constructions are not described in detail to avoid obscuring the present disclosure in unnecessary detail. Therefore, specific structural and functional details disclosed herein are not to be interpreted as limiting. Like reference numerals refer to similar or identical elements throughout the description of the figures.

As used herein, the phrase "electrosurgical system" refers to any system that imparts energy (e.g., electrical, ohmic, resistive, ultrasonic, microwave, cryogenic, laser .) to tissue to achieve a desired effect. The term "reposable instrument" refers to an instrument that has a limited number of uses or an instrument that includes a disposable portion and a reusable portion wherein the disposable portion has a limited number of uses. The phrase "end effector" refers to the portion of the electrosurgical system that imparts energy to tissue. The phrase "transmission line" generally refers to any transmission medium that can be used for the propagation of signals from one point to another.

The generator according to the present disclosure can perform ablation, monopolar and bipolar electrosurgical procedures and ultrasonic electrosurgical procedures. The generator may include a plurality of outputs for interfacing with various electrosurgical instruments (e.g., a monopolar active electrode, return electrode, bipolar jaw members, footswitch, ultrasonic horn). Further, the generator includes electronic circuitry configured for generating power specifically suited for various electrosurgical modes (e.g., cutting, blending, division, fragmenting, coagulating) and procedures.

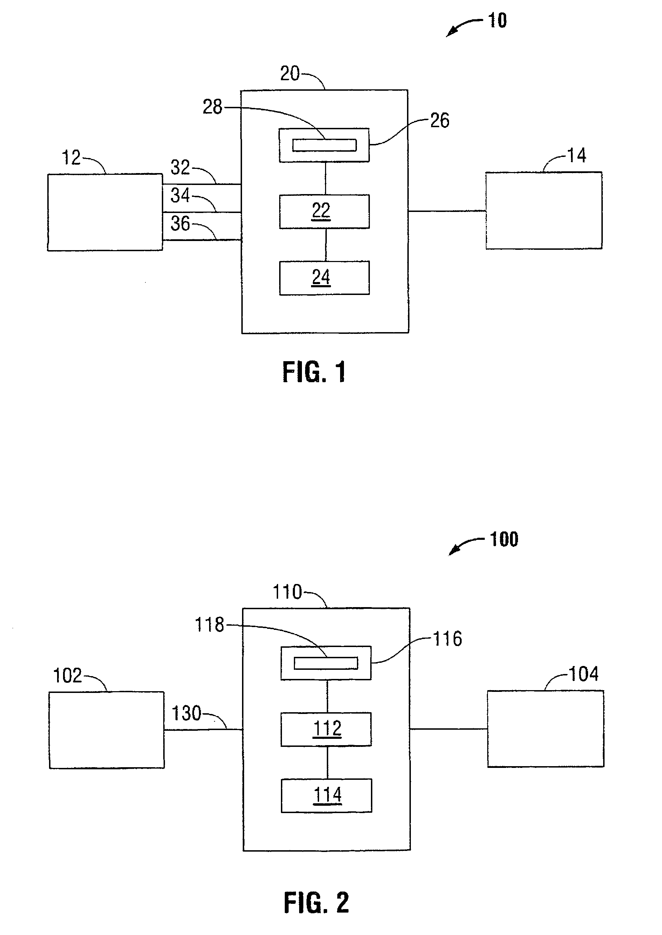

Turning to Fig. 1, an electrosurgical system is shown generally as 10. Electrosurgical system 10 includes a generator 12 that delivers energy to a reposable instrument 20 via transmission line 32. The generator 12 includes suitable input controls (e.g., buttons, activators, switches, touch screen ) for controlling the generator 12. In addition, the generator 12 may include one or more display screens for providing the user with a variety of output information (e.g., intensity settings, treatment complete indicators). The controls allow the user to adjust power of the energy, waveform, as well as the level of maximum arc energy allowed that varies depending on desired tissue effects and other parameters to achieve the desired waveform suitable for a particular task (e.g., coagulating, tissue sealing, intensity setting). The reposable instrument 20 may also include a plurality of input controls that may be redundant with certain input controls of the generator 12. Placing the input controls at the reposable instrument 20 allows for easier and faster modification of energy parameters during the surgical procedure without requiring interaction with the generator 12.

Reposable instrument 20 is a handheld device that receives energy from generator 12 and imparts energy to tissue via an end effector 14 coupled thereto. End effector 14 may be a monopolar active electrode, bipolar jaw members or an ultrasonic horn. Reposable instrument 20 includes a controller 22, memory 24 and a timing device 26. Controller 22 may be a microprocessor operably connected to memory 24 and timing device 26. Those skilled in the art will appreciate that the microprocessor may be substituted by any logic processor or analog circuitry (e.g., control circuit) adapted to perform the functions discussed herein. Memory 24 may be a volatile type memory (e.g., RAM) and/or non-volatile type memory (e.g., Phase Change Memory, PRAM, EEPROM, flash media, disk media ).

Timing device 26 tracks usage of the reposable instrument 20 and/or end effector 14 using a built in counter 28 and provides the information to controller 22. Timing device 26 may be a mechanical timer, an electrical timer composed of discrete digital and analog components or timing device 26 may be implemented as software in a microprocessor. Controller 22. memory 24 and timing device 26 may be individual components operatively coupled to each other or they may be included in a single integrated circuit.

During operation of electrosurgical system 10, end effector 14 is operatively coupled to reposable instrument 20 and reposable instrument 20 is operatively coupled to generator 12. Energy is delivered from generator 12 to reposable instrument 20 via transmission line 32. In one embodiment, when generator 12 supplies energy to reposable instrument 20, a timing signal is sent via transmission line 34 to reposable instrument 20. The timing signal could be sent via a discrete connection such as transmission line 34 or provided over a serial communication path. The timing signal is static during inactivation or idle and provides a waveform during activation. The timing signal can be in any frequency or duration, such as 1Hz or 1KHz, which can be used to adequately track usage of the reposable instrument. The finer the resolution, the larger the accumulated value and resulting storage space required.

Reposable instrument 20 receives the timing signal and timing device 26 increments counter 28. The counter 28 may indicate the number of times reposable instrument 20 has been used, amount of energy delivered to reposable instrument 20 or by reposable instrument 20, the number of times end effector 14 has been used, amount of energy delivered by end effector 14 or amount of time the generator delivers energy to the reposable instrument 20. Timing device 26 provides counter information from counter 28 to controller 22 and controller 22 stores the counter information in memory 24 as usage history. When the usage history exceeds a predetermined limit, controller 22 disables reposable instrument 20 or end effector 14 via software or hardware, such as turning off a switch, so that reposable instrument 20 or end effector 14 can not be used to deliver energy. Exceeding the predetermined limit indicates that the reposable instrument 20 or end effector 14 has reached its lifespan and a new reposable instrument or end effector should be used. Furthermore, reposable instrument 20 may also include an indicator (not shown) such as a light or audible sound to indicate that the reposable instrument 20 or end effector 14 has reached the end of their respective lifespans. The usage history stored in memory 24 may be reset by the generator 12 or reposable instrument 20 when a new, repaired or refurbished reposable instrument is attached to the generator 12.

A transmission line 36 may also be coupled between generator 12 and reposable instrument 20. Transmission line 36 may be a data line that transmits data between reposable instrument 20 and generator 12. Such data may include a device identifier for the generator 12, a device identifier for the reposable instrument 20, type of reposable instrument 20 being used, type of end effector 14 being used, type of electrosurgical generator 12 (e.g., ablation, electrosurgical, ultrasonic ), number of times the reposable instrument 20 has been used, number of times the end effector 14 has been used, amount of energy delivered by the reposable instrument 20, amount of energy delivered by the end effector 14, amount of time the reposable instrument 20 has been coupled to the generator 12, whether the reposable instrument 20 has reached the end of the reposable instrument's 20 lifespan, whether the end effector 14 has reached the end of the end effector's 14 lifespan or readings from any sensors (not shown) disposed on the reposable instrument 20 or end effector 14. Reposable instrument 20 may also transmit a signal to generator 12 to discontinue transmitting energy via transmission line 32.

Although Fig. 1 shows three separate transmission lines 32, 34 and 36, system 10 may utilize a single conduit having transmission lines 32, 34 and 36 included therein. Alternatively, transmission lines 34 and 36 may be combined into a single transmission line and the timing signal and data being transmitted may be multiplexed onto the single transmission line.

Typically, generator 12 would track reposable instrument's 20 usage during electrosurgical applications. As a result, reposable instrument 20 had to be paired with a specific generator. If reposable instrument 20 was paired with a different generator 12, the reposable instrument 20 usage time, as measured by the generator 12, would lead to inaccurate usage history that may result in damage to the electrosurgical system 10 components. By placing timing device 26 in reposable instrument 20, reposable instrument 20 can be paired with any generator 12 while still maintaining an accurate usage history. Accordingly, when the usage history indicates that reposable instrument 20 has reached the end of its lifespan, controller 22 disables reposable instrument 20 or transmits a signal to the generator 12 to cease energy delivery.

Turning to Fig. 2, another electrosurgical system is shown generally as 100. System 100 includes a generator 102 and end effector 104 similar to generator 12 and end effector 14 of Fig. 1. System 100 also has a reposable instrument 110 that includes a controller 112, memory 114 and timing device 116 somewhat similar to the controller 22, memory 24 and timing device 26 of Fig. 1.

Unlike electrosurgical system 10 of Fig. 1, electrosurgical system 100 does not require a timing signal from generator 102. When energy is delivered from generator 102 to reposable instrument 110 via transmission line 130, generator 102 or controller 112 may provide an activation waveform, ON command or other signal to timing device 116 to increment counter 118. If counter 118 keeps track of the number of times the reposable instrument 110 is used, the counter 118 is only incremented by one. If counter 118 keeps track of the amount of energy being delivered by generator 102 or reposable instrument 110, then the counter 118 keeps incrementing until energy is no longer being delivered by generator 102 or the reposable instrument 110 or timing device receives an OFF command. Timing device 116 provides counter information from counter 118 to controller 112. Controller 112 stores the counter information in memory 114 as usage history and compares the usage history to a predetermined limit. If the counter information exceeds the predetermined limit, controller 112 disables reposable instrument 110 or end effector 104 via software or hardware, so that reposable instrument 110 or end effector 104 can not be used to deliver energy.

System 100 does not rely on an external timing signal to track usage of reposable instrument 110. This may be advantageous when using reposable instrument 110 with different generators because the timing signal provided by each separate generator may be out of sync thereby leading to inaccuracies in the usage history for reposable instrument 110.

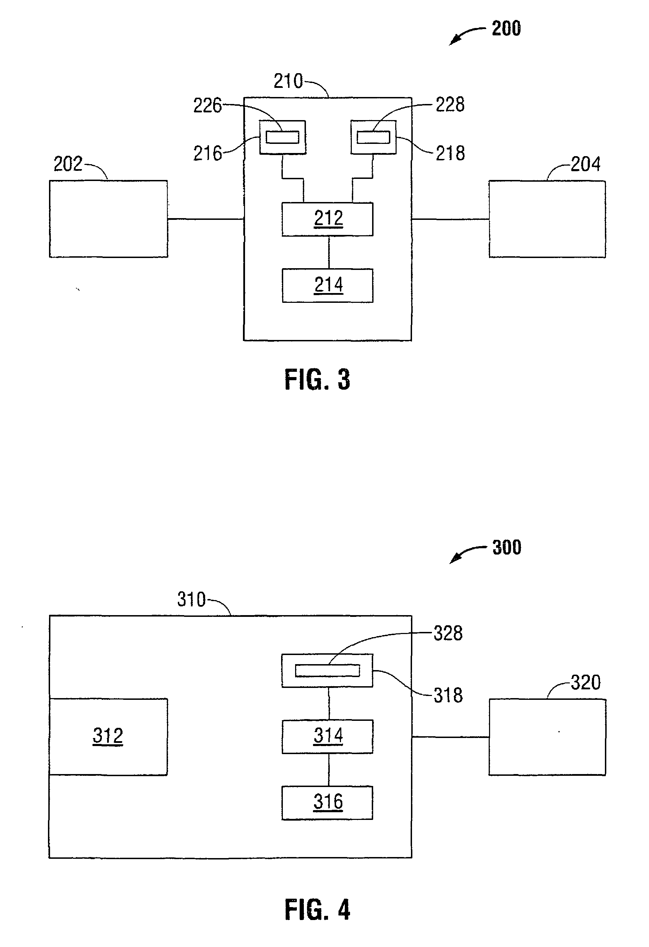

Turning to Fig. 3, another electrosurgical system is shown generally as 200. System 200 includes a generator 202 and end effector 204 similar to generator 12 and end effector 14 of Fig. 1. System 200 also has a reposable instrument 210 that includes a controller 212 and memory 214 somewhat similar to the controller 22 and memory 24 of Fig. 1.

In some electrosurgical systems, the reposable instrument 210 may have a different window of use than the end effector. For instance, end effector 204 may have a smaller window of use than reposable instrument 210 so that many end effectors 204 may be used with a single reposable instrument 210. Accordingly, reposable instrument 210 includes two timing devices 216 and 218 where each timing device is similar to the timing devices described above. Timing device 216 is configured to may track the usage history of reposable instrument 210 while timing device 218 is configured to track the usage of end effector 204.

Timing device 216 transmits reposable instrument 210 counter information obtained from counter 226 to controller 212 and controller 212 stores the reposable instrument 210 counter information in memory 214 as reposable instrument 210 usage history. Controller 212 compares the reposable instrument usage history to a predetermined limit and if the reposable instrument 210 usage history exceeds the predetermined limit, reposable instrument 210 is disabled from delivering energy. Furthermore, reposable instrument 210 may provide a visual or audible indication that reposable instrument 210 has exceeded its window of use.

When end effector 204 is inserted into reposable instrument 210, timing device 218 resets and starts incrementing counter 228 when energy is delivered by end effector 204. Timing device 218 transmits end effector counter information to controller 212 and controller 212 stores the end effector counter information in memory 214 as end effector usage history. Controller 212 compares the end effector 204 usage history to a predetermined limit and if the end effector 204 usage history exceeds the predetermined limit, end effector 204 is disabled from delivering energy. Furthermore, reposable instrument 210 may provide a visual or audible indication that end effector 204 has exceeded its window of use.

Turning now to Fig. 4, a portable electrosurgical system is shown generally as 300. System 300 includes a reposable instrument 310 and an end effector 320. Reposable instrument 310 includes a generator 312 that may be inserted into reposable instrument 310. Generator 312 includes a power supply or battery and energy source that may output ultrasonic energy, radio frequency energy or microwave energy. Reposable instrument 310 also includes a controller 314, memory 315 and a timing device 318.

Timing device 318 tracks usage of generator 312, reposable instrument 310 and/or end effector 320 using a built in counter 328 and provides the information to controller 314. Timing device 318 may be a mechanical timer, an electrical timer composed of discrete digital and analog components or timing device 318 may be implemented as software in a microprocessor.

During operation of electrosurgical system 300, end effector 304 is operatively coupled to reposable instrument 310 and reposable instrument 310 is operatively coupled to generator 312. When energy is delivered by reposable instrument 310, timing device 318 increments counter 328. Counter 328 may indicate the number of times reposable instrument 310 has been used, amount of energy delivered to reposable instrument 310 or by reposable instrument 310, the number of times end effector 304 has been used, amount of energy delivered by end effector 304, or the amount of energy outputted by generator 312. Timing device 318 provides counter information from counter 328 to controller 314 which stores the counter information in memory 316 as usage history. When the usage history exceeds a predetermined limit, controller 314 disables reposable instrument 310 or end effector 304 via software or hardware, so that reposable instrument 310 or end effector 14 can not be used to deliver energy. Furthermore, reposable instrument 310 may also include a visual or audible indicator to indicate that the reposable instrument 310 or end effector 304 has reached the end of their respective lifespans. When the reposable instrument 310 needs to be replaced, generator 312 is removed from reposable instrument 310 and inserted into a new reposable instrument.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. The claims can encompass embodiments in hardware, software, or a combination thereof.

## Claims

1. A hand-held reposable instrument (210) for use with an electrosurgical system (200), the reposable instrument comprising:
a reposable instrument timing device (216) configured to track usage of the reposable instrument;
a controller (212) configured to control operation of the electrosurgical system based on the usage of the reposable instrument; and
a memory (214) configured to store information pertaining to the usage of the reposable instrument,
wherein the reposable instrument timing device is configured to provide reposable instrument usage information to the controller and the controller is configured to store the reposable instrument usage information in the memory,
wherein the controller compares the reposable instrument usage information stored in the memory with a predetermined limit, and **characterized in that**:
the controller is configured to disable the reposable instrument from delivering energy if the reposable instrument usage information exceeds the predetermined limit;
the reposable instrument further comprising an end effector timing device (218) configured to track usage of an end effector coupleable to the reposable instrument,
wherein the end effector timing device is configured to provide end effector usage information to the controller and the controller is configured to store the end effector usage information in the memory; and
the controller is configured to compare the end effector usage information stored in the memory with a predetermined limit and the controller is configured to disable the end effector from delivering energy if the end effector usage information exceeds the predetermined limit.

2. The reposable instrument according to claim 1, wherein the controller is configured to compare the reposable instrument usage information stored in the memory with a predetermined limit and the controller is configured to provide a visual or audible indication to a user if the reposable instrument usage information exceeds the predetermined limit.

3. The reposable instrument according to claim 1 or 2, wherein the controller is configured to compare the end effector usage information stored in the memory with a predetermined limit and the controller is configured to provide a visual or audible indication to a user if the end effector usage information exceeds the predetermined limit.

4. An electrosurgical system (200), comprising:
a generator (202) configured to output energy to a reposable instrument (210);
an end effector (204) operatively configured to output to tissue energy received from the reposable instrument; and
the reposable instrument as claimed in any one of the preceding claims and operatively coupleable between the generator and the end effector.

5. The electrosurgical system according to claim 4, further comprising a transmission line (34; 130) configured to provide a timing signal from the generator to the reposable instrument.

6. The electrosurgical system according to claim 4 or 5, further comprising a transmission line (36; 130) configured to transfer data between the generator and the reposable instrument.

## Patentansprüche

1. Wiederverwendbares Handinstrument (210) zur Verwendung mit einem elektrochirurgischen System (200), das wiederverwendbare Instrument mit:
einer Zeiteinstellungseinrichtung (216) des wiederverwendbaren Instruments, die eingerichtet ist, die Verwendung des wiederverwendbaren Instruments zu verfolgen;
einem Regler (212), der eingerichtet ist, den Betrieb des elektrochirurgischen Systems auf Grundlage der Verwendung des wiederverwendbaren Instruments zu steuern; und
einem Speicher (214), der eingerichtet ist, eine die Verwendung des wiederverwendbaren Instruments betreffende Information zu speichern,
wobei die Zeiteinstellungseinrichtung des wiederverwendbaren Instruments eingerichtet ist, dem Regler eine Verwendungsinformation des wiederverwendbaren Instruments bereitzustellen und der Regler eingerichtet ist, die Verwendungsinformation des wiederverwendbaren Instruments in dem Speicher zu speichern, und
der Regler die in dem Speicher gespeicherten Verwendungsinformationen des wiederverwendbaren Instruments mit einem festgelegten Grenzwert vergleicht, **dadurch gekennzeichnet**, das:
der Regler eingerichtet ist, die Energiezufuhr von dem wiederverwendbaren Instrument zu deaktivieren, wenn die Verwendungsinformation des wiederverwendbaren Instruments den festgelegten Grenzwert überschreitet;
wobei das wiederverwendbare Instrument des Weiteren eine Endeffektorzeiteinstellungseinrichtung (218) aufweist, die eingerichtet ist, die Verwendung eines Endeffektors zu verfolgen, der mit dem wiederverwendbaren Instrument koppelbar ist;
die Endeffektorzeiteinstellungseinrichtung eingerichtet ist, dem Regler eine Endeffektorverwendungsinformation bereitzustellen und der Regler eingerichtet ist, die Endeffektorverwendungsinformation in dem Speicher zu speichern; und
der Regler eingerichtet ist, die in dem Speicher gespeicherte Endeffektorverwendungsinformation mit einem festgelegten Grenzwert zu vergleichen und die Energiezufuhr von dem Endeffektor zu deaktivieren, wenn die Endeffektorverwendungsinformation den festgelegten Grenzwert überschreitet.

2. Wiederverwendbares Instrument nach Anspruch 1, bei dem der Regler eingerichtet ist, die in dem Speicher gespeicherte Verwendungsinformation des wiederverwendbaren Instruments mit einem festgelegten Grenzwert zu vergleichen und einem Verwender einen visuellen oder hörbaren Hinweis bereitzustellen, wenn die Verwendungsinformation des wiederverwendbaren Instruments den festgelegten Grenzwert überschreitet.

3. Wiederverwendbares Instrument nach Anspruch 1 oder 2, bei dem der Regler eingerichtet ist, die in dem Speicher gespeicherte Verwendungsinformation des Endeffektors mit einem festgelegten Grenzwert zu vergleichen und einem Verwender einen visuellen oder hörbaren Hinweis bereitzustellen, wenn die Verwendungsinformation des Endeffektors den festgelegten Grenzwert überschreitet.

4. Elektrochirurgisches System (200), mit:
einem Generator (202), der eingerichtet ist, einem wiederverwendbaren Instrument (210) Energie auszugeben;
einem Endeffektor (204), der eingerichtet ist, im Betrieb die von dem wiederverwendbaren Instrument empfangene Energie an Gewebe auszugeben; und
dem wiederverwendbaren Instrument gemäß einem der vorstehenden Ansprüche und betriebsfähig zwischen dem Generator und dem Endeffektor koppelbar.

5. Elektrochirurgisches System nach Anspruch 4, des Weiteren mit einer Übertragungsleitung (34; 130), die eingerichtet ist, dem wiederverwendbaren Instrument ein Zeiteinstellungssignal von dem Generator bereitzustellen.

6. Elektrochirurgisches System nach Anspruch 4 oder 5, des Weiteren mit einer Übertragungsleitung (36; 130), die eingerichtet ist, Daten zwischen dem Generator und dem wiederverwendbaren Instrument zu übertragen.

## Revendications

1. Instrument reposable tenu à la main (210) pour utilisation avec un système électro-chirurgical (200), l'instrument reposable comprenant :
un dispositif d'horloge (216) d'instrument reposable configuré pour suivre l'utilisation de l'instrument reposable ;
un dispositif de commande (212) configuré pour commander le fonctionnement du système électro-chirurgical sur la base de l'utilisation de l'instrument reposable ; et
une mémoire (214) configurée pour stocker des informations se rapportant à l'utilisation de l'instrument reposable,
où le dispositif d'horloge (216) d'instrument reposable est configuré pour fournir des informations d'utilisation de l'instrument reposable au dispositif de commande, et le dispositif de commande est configuré pour stocker les informations d'utilisation de l'instrument reposable dans la mémoire,
où le dispositif de commande compare les informations d'utilisation de l'instrument reposable stockées dans la mémoire avec une limite prédéterminée, et **caractérisé en ce que** :
le dispositif de commande est configuré pour empêcher l'instrument reposable de délivrer de l'énergie si les informations d'utilisation de l'instrument reposable dépassent la limite prédéterminée ;
l'instrument reposable comprenant en outre un dispositif d'horloge d'effecteur terminal (218) configuré pour suivre l'utilisation d'un effecteur terminal apte à être couplé à l'instrument reposable ;
où le dispositif d'horloge d'effecteur terminal est configuré pour fournir les informations d'utilisation de l'effecteur terminal au dispositif de commande, et le dispositif de commande est configuré pour stocker les informations d'utilisation de l'effecteur terminal dans la mémoire ; et
le dispositif de commande est configuré pour comparer les informations d'utilisation de l'effecteur terminal stockées dans la mémoire avec une limite prédéterminée, et le dispositif de commande est configuré pour empêcher l'effecteur terminal de délivrer de l'énergie si les informations d'utilisation de l'effecteur terminal dépassent la limite prédéterminée.

2. Instrument reposable selon la revendication 1, dans lequel le dispositif de commande est configuré pour comparer les informations d'utilisation de l'instrument reposable stockées dans la mémoire avec une limite prédéterminée, et le dispositif de commande est configuré pour fournir une indication visuelle ou audible à un utilisateur si les informations d'utilisation de l'instrument reposable dépassent la limite prédéterminée.

3. Instrument reposable selon la revendication 1 ou 2, dans lequel le dispositif de commande est configuré pour comparer les informations d'utilisation de l'effecteur terminal stockées dans la mémoire avec une limite prédéterminée, et le dispositif de commande est configuré pour fournir une indication visuelle ou audible à un utilisateur si les informations d'utilisation de l'effecteur terminal dépassent la limite prédéterminée.

4. Système électro-chirurgical (200), comprenant :
un générateur (202 configuré pour émettre de l'énergie à un instrument reposable (210) ;
un effecteur terminal (204) fonctionnellement configuré pour émettre au tissu l'énergie reçue de l'instrument reposable ; et
l'instrument reposable tel que revendiqué dans l'une quelconque des revendications précédentes et apte à être fonctionnellement couplé entre le générateur et l'effecteur terminal.

5. Système électro-chirurgical selon la revendication 4, comprenant en outre une ligne de transmission (34 ; 130) configurée pour fournir un signal d'horloge du générateur à l'instrument reposable.

6. Système électro-chirurgical selon la revendication 4 ou 5, comprenant en outre une ligne de transmission (36 ; 130) configurée pour transférer des données entre le générateur et l'instrument reposable.
